# EUROPEAN PATENT SPECIFICATION

(11) **EP 0 311 388 B1**
(45) Date of publication and mention of the grant of the patent: **22.02.1995**
(21) Application number: 88309300.7
(22) Date of filing: 06.10.1988
(51) Int. Cl.: C12Q 1/68

(54) **A rapid method for identifying a specific nucleic acid sequence**
Schnelles Verfahren zur Identifizierung einer spezifischen Sequenz von Nukleinsäure
Procédé rapide pour identifier une séquence spécifique d'acide nucléique

(30) Priority: 06.10.1987 GB 8723445; 30.09.1988 US 250703
(43) Date of publication of application: 12.04.1989
(73) Proprietor: ORTHO DIAGNOSTIC SYSTEMS INC., Raritan New Jersey 08869 (US)
(72) Inventor: Botsko, Eleanor, Chester, N.J. 07930 (US); Woods, Derek E., Flemington, N.J. 08822 (US); Kochesky, Richard, Bloomsbury, N.J. 08804 (US)
(74) Representative: Mercer, Christopher Paul

(56) References cited:
- EP-A- 0 228 075
- EP-A- 0 231 010
- EP-A- 0 237 737
- EP-A- 0 357 437
- WO-A-86/00139
- ABSTRACTS, ANNUAL MEETING OF THE AMERICAN SOCIETY FOR MICROBIOLOGY, vol. 87, no. 0, March 1987, Washington, DC (US); D.K. BUXTON et al., p. 369#
- METHODS IN ENZYMOLOGY, vol. 152, 1987, New York, NY (US); L.M. ANGERER et al., pp. 649-660#
- BIOSIS, 1987; G.I. CARTER et al., no. 85005586#
- ANTIMICROBIAL AGENTS & CHEMOTHERAPY, vol. 28, no. 1, July 1985; T.D. GOOTZ et al., pp. 69-73#

## Description

The present invention concerns a rapid nucleic acid hybridization method for identifying a specific nucleic acid sequence, especially a sequence specific for a microorganism. The method uses very low volumes of a concentrated probe reagent, and is useful for research, test confirmation, and diagnostic purposes.

The genetic information of all organisms is stored in specialized molecules called DNA. The unique structure of DNA was first described by Watson and Crick in 1953. The DNA molecule consists of two linear polymeric strands, intertwined to form a double helix. Each strand is composed of alternating sugar and phosphate groups, stabilized by hydrogen bonding between pairs of nucleotide bases: adenine (A), guanine (G), cytosine (C), and thymine (T). The two strands are complementary, with A always bonding to T on the opposite strand and G always bonding to C. Thus, if the sequence of bases in one strand is known, the sequence of its partner is also known. The sugar and phosphate groups maintain the structural integrity of the DNA molecule and remain constant throughout the molecule. Only the number of base pairs and the sequence in which the base pairs occur will vary. It is the precise number and sequence of bases along the strand which encodes all genetic information. Hence, the physical and biological nature of all organisms is determined by the unique sequence of nucleotide bases in its DNA. The identity of an organism can be determined, and even minor differences between related organisms can be detected if the sequence of bases responsible is known and can be observed.

The double-stranded DNA molecule is normally very stable; however, the two complementary strands can be separated (denatured). In vivo, natural processes such as DNA replication (synthesis of identical "daughter" molecules) and transcription (synthesis of messenger RNA) require that the two strands separate and serve as templates. In vitro, DNA can be denatured by treatment with heat or extremes of pH. These simple procedures break the hydrogen bonds which hold the complementary pairs together while preserving the integrity of the base sequences in the relatively stable single-stranded DNA. Under appropriate conditions, because of the nucleotide complementarity and hydrogen bonding, the single strands will rewind (reanneal). This process in which a double-stranded molecule is formed by specific sequence-dependent interaction of complementary single strands is called nucleic acid hybridization.

Nucleic acid probes are specific, single-stranded, nucleic acid sequences that search for complementary sequences in a pool of single-stranded nucleic acid. Under proper conditions, the complementary strands collide, complementary sequences recognize each other and form double-stranded molecules. The nucleic acid probe can be labelled using either a radioisotope or one of several nonradioactive labels, thereby allowing visualization of the nucleic acid hybridization reaction.

Nucleic acid probe hybridization techniques have long been a tool of molecular biologists. Recent improvements in nucleic acid technology have now expanded the use of these probes to the clinical setting. Possible applications of this technology include direct and indirect diagnosis of both infectious and genetically inherited diseases. The clinical microbiology lab currently utilizes classical biochemical reactions and antibody-based agglutination techniques to identify pathogens. These methods are often expensive, cumbersome and time-consuming. Nucleic acid probe technology in the clinical microbiology lab promises to be cost effective, rapid and simple. Further advantages of this technology include the ability to detect the presence of the target organism regardless of its metabolic state, the ability to detect either broad or narrow groups of related species, and the potential for developing a single rapid procedure that uses the same basic format for all tests.

The Reexam of U.S. Patent No. 4,358,535 (Falkow et al.) teaches a method for detecting the presence of a pathogen in a clinical sample utilizing nucleic acid hybridization techniques. The method involves depositing a clinical sample consisting essentially of physiological fluid or excreta, on an inert support, such as a filter paper, and then treating the sample so as to fix genetic material from the pathogen to the support in substantially single-stranded form. The filter paper with the fixed single-stranded genetic material is then introduced into a labeled probe solution, where the probe is complementary to the genetic material and hybridizes with the genetic material.

The Falkow et al. method has the disadvantages of requiring a time consuming and tedious protocol. The time required for the hybridization step alone is in the order of 24 hours and the time required for the entire assay is 48 hours or more. The Falkow et al. method requires that large volumes of probe solution and other solutions must be used because the filter having the fixed DNA must be dipped into the probe solution. This requires 1-10 milliliters of probe solution. As the probe solution is the most expensive reagent used in the method, use of large volumes of probe reagent significantly increases the cost of performing the method and any commercial product based thereon.

The present method avoids the disadvantages of the Falkow et al. method by performing steps of the method directly on an isolated reaction surface. The reaction surface need not be dipped into probe reagent. Rather, low volumes (approximately 2-30 microliters) of these reagents and solutions are placed directly on the reaction surface. Prehybridization steps and numerous washing steps are no longer required, as may ordinarily be expected in the art. Thus, the method provides substantial savings on cost of reagents and time. Most importantly, the method provides for rapid results because a very high concentration of probe reagent can be used (at low cost) to drive the hybridization reaction to completion. Results may be obtained in under 10 minutes.

Gootz et al., in Antimicrobial Agents and Chemotherapy, 28, 1, 69-73, 1985, describe a spot DNA hybridization assay for identifying and following the dissemination of specific aminoglycoside resistance genes in clinical isolates of bacteria. The assay described by Gootz takes a minimum of 66 hours to complete.

The present invention provides, in a first aspect, a method, for identifying whether a sample contains a nucleic acid sequence specific for a particular microorganism, comprising:
a) applying a limited amount of said sample to an isolated nucleic acid binding surface having a width or diameter of from 2 mm to 5.0 mm, which is attached to an inert support;
b) treating said sample with a lysis reagent so as to release nucleic acids and so as to bind said nucleic acids to said support in substantially single-stranded form;
c) applying from 5 »l to 30 »l of a reagent containing at least one detectably-labeled nucleic acid probe, said probe being present in said reagent at a concentration of from 0.2 to 5,000 pM/ml, directly onto said binding surface under hybridization conditions, for a length of time appropriate to allow hybridization to take place;
d) washing said binding surface; and
e) detecting hybridization of the probe with the nucleic acid on the binding surface by means of the detectable label, thereby identifying whether said specific nucleic acid sequence is present.

In a second aspect, the present invention also provides a device for carrying out the method described above comprising:
a) a substantially rigid inert support; and
b) a plurality of isolated nucleic acid binding surfaces having a width or diameter of from 2 mm to 5 mm, said surfaces being adherently attached to a surface of the support and being separated from one another by at least 1 mm.

In a third aspect, the present invention further provides a kit for carrying out the method described above comprising:
a) a substantially rigid inert support;
b) a plurality of isolated nucleic acid binding surfaces having a width or diameter of from 2mm to 5 mm, said surfaces being adherently attached to a surface of the support and being separated from one another by at least 1 mm; and
c) a vessel containing a detectably labeled nucleic acid probe reagent, said probe being present in said reagent at a concentration of from 0.2 to 5,000 pM/ml, wherein the probe is specific for a microorganism to be identified and has a nucleotide sequence complementary to at least a portion of the nucleic acid within the microorganism.

Preferred features of the invention are recited in the dependent claims.

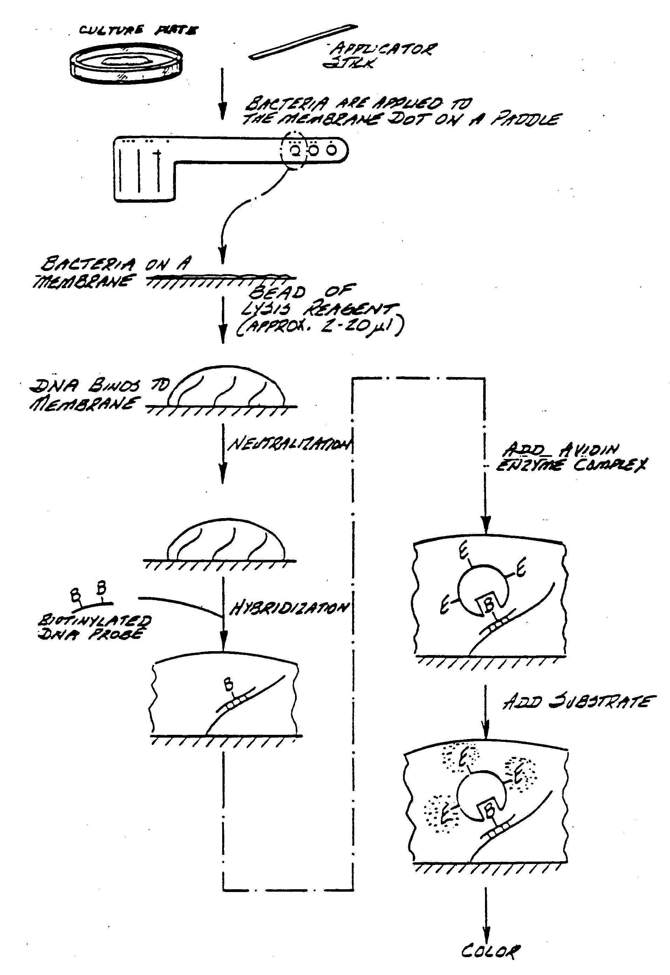

The Figure depicts a preferred embodiment of the assay method of the invention and a device having three isolated nucleic acid binding surfaces.

The nucleic acid hybridization reaction is concentration dependent and is normally a second-order reaction. A second order reaction is one in which the rate of reaction is proportional to the product of the concentrations of two reactants or to the second power of a single reactant. In the nucleic acid hybridization reaction, the reaction rate is directly proportional to the product of the concentrations of the two nucleic acid sequences to be hybridized.

The amount of nucleic acid present in a sample, especially a sample of a microorganism, may be relatively low. Therefore, the rate of a hybridization reaction to detect this nucleic acid will be low. However, use of a very high concentration of the probe reagent can make the reaction a pseudo-first-order reaction, and thus significantly increase the reaction rate. A first-order reaction is one in which the reaction rate is exactly proportional to the concentration of one reactant. By using an excess concentration of probe reagent, the hybridization reaction can be driven to completion within minutes rather than hours as in prior methods. When an excess of the probe reagent is used, its concentration remains essentially unchanged throughout the reaction.

The present invention provides a cost-effective method of using a high concentration of probe reagent to obtain a high rate of reaction. Heretofore, it was not economically feasible to use high concentrations of probe reagents because of the relatively large volumes required and high cost of the probes. Prior methods required about 1-5 milliliters of probe reagent solution because the filters on which the hybridization reaction took place were dipped into the probe reagent. For example, in a preferred embodiment, the present method may be conducted with a probe solution having a concentration of 0.02 picomoles/microliter. Spotting 10 microliters of such a reagent onto the nucleic acid binding surface would then deposit 0.2 picomoles of probe onto the surface. If the nucleic acid binding surface were dipped, as in the prior methods, then at least one milliliter of probe solution would be required. If the same concentration were used as in the present method, then 20 picomoles of probe would be required. This is 100 times the amount of probe required for the present method. As the probe reagent is an expensive reagent used in a nucleic acid hybridization assay, it is readily apparent that the present method provides significant cost savings.

The method of the present invention is conducted on a surface rather than in a container. An "isolated nucleic acid binding surface" attached to a substantially rigid support is used as the hybridization reaction zone. As used herein, "isolated nucleic acid binding surface" refers to a discrete zone of material capable of binding nucleic acids. One or more of said binding surfaces are affixed onto a support, separate and apart from any other surfaces present on the support. The binding surface may take any shape, but is preferred to be circular. The binding surface has a small surface area and it is relatively small in size, for example, on the order of the size of a drop of aqueous fluid. If circular, the binding surface may preferably have a diameter of about 1-5 millimeters, and may be referred to as a "dot", and if rectangular or square, a width of about 1-5 millimeters. Use of an isolated nucleic acid binding surface provides a reaction zone having a small surface area. This permits the target nucleic acid sequence to be in direct contact with all reagents and solutions spotted onto the zone. However, it should be appreciated that the binding surface may be dipped into inexpensive lysis and wash solutions, rather then spoting these solutions onto the surface. Use of isolated binding surfaces also prevents cross-contamination, provided sample and reagents are added to the surface in an amount not to exceed its boundaries, as taught herein. Accordingly, many different samples may be tested in the same assay for the presence of a particular target nucleic acid. Samples that are positive for a particular nucleic acid may be positioned right next to those that are not. Conversely, various different probe reagents may be spotted onto the individual binding surfaces, each containing the same sample, to test for the presence of various different nucleic acids in a given sample.

The limited or restricted volume of probe reagent used in the present method may be within the range of about 1-30 microliter aliquots. Preferably, the limited volume is within the range of about 5-20 microliters for a binding surface of about 2 mm - 5 mm. The concentration of the probe in the reagent may be within the range of about 0.2-5000 picomoles/milliliters. Preferably, the concentration is about 2.0-200 picomoles/milliliter.

The detectable label may be any ligand or hapten which is capable of being detected, directly or indirectly, and thereby indicate that hybridization has occurred. The label is preferred to be an enzyme capable of causing a color change in a substrate solution or a ligand capable of binding to such an enzyme. It should be appreciated that suitable enzymes are those such as horseradish peroxidase (HRP) and alkaline phosphatase. A suitable ligand might be a hapten such as fluorescein, iminobiotin, and biotin which is capable of binding to an avidin or streptavidin enzyme complex. When a biotin-labeled probe is used, an avidin or streptavidin enzyme complex is applied to the binding surface and then an enzyme substrate solution is added to the surface. Detection of a color change of the surface indicates that hybridization has occurred. Alternatively, the label may be a radioactive molecule such as ³²P, ¹²⁵I or ³H; a fluorescent molecule, such as fluorescein and rhodamine; a chemiluminescent molecule such as luciferin and luminol; or a light scattering particle, such as colloidal gold. Suitable detection methods are scintillation counting, autoradiography, fluorescence measurement, colormetric measurement, light emission and the like.

The nucleic acid sequence to be identified may be either DNA or RNA. The probe should have at least 15 bases to ensure efficient hybridization, and it will usually have about 15-100 bases. The probe may be prepared by organic synthesis, recombinant DNA techniques or isolation from genomic nucleic acids.

Microorganisms capable of being identified by the present method are bacteria, including pathogens; viruses; fungi, such as yeast and molds; and protozoa. The nucleic acid from the microorganism to be identified is characteristic of the specific organism from which it is derived. Thus, detection of the specific nucleic acid sequence corresponds to detection of the organism in the sample tested.

In the first step of the method of the invention, a sample, containing the nucleic acid to be detected, is placed on the nucleic acid binding surface by dabbing a small amount onto the surface, approximately within its boundaries. In some cases the nucleic acid may be present in solution and the amount applied to the nucleic acid binding surface is simply allowed to dry. Care should be taken that the amount applied does not overflow the boundaries of the binding surface.

In other embodiments, the target nucleic acid sequence (i.e. the sequence which is to hybridize with the probe) must be removed from an organism containing the sequence, such as a cell, and the sequence is then be provided in single-stranded form. The target sequence is removed from an organism containing it by lysis of the organism with a suitable detergent or proteolytic and/or hydrolytic enzyme. If need be, single-stranded form can be achieved by denaturing the DNA using standard techniques, such as exposure to heat, extremes of pH, decrease in the dielectric constant of the aqueous medium or exposure to urea, amides or similar solutes. Denaturation is the separation of complementary strands of double-stranded nucleic acid. All or part of the nucleic acid molecule may be denatured, as long as amounts sufficient to allow hybridization with the probe are provided. If the nucleic acid is already in substantially single-stranded form, such as single stranded ribosomal, messenger and transfer RNA's, then a specific denaturating step is not required. Preferably, this cell lysis and/or denaturation is achieved by contacting the nucleic acid with dilute aqueous alkali (e.g., 0.1-1.0 M NaOH) which can lyse cells as well as denature nucleic acid. It is preferable to drop such a lysis solution onto the binding surface in such a manner and an amount that will fit approximately within its boundaries. Any excess lysis reagent may be conveniently removed by simply blotting the area surrounding the binding surfaces. The binding surface may then be neutralized using conventional reagents.

No pre-hybridization step to block non-specific binding (such as with the use of Denhardt's solution, dextran sulfate, BSA and the like) is required with the method of the invention. While the present inventors do not wish to be bound by theory, they believe that the above-mentioned addition of a lysis reagent to the boundaries of the binding surfaces accomplishes both cellular lysis and the blocking of non-specific binding sites, possibly with the cellular debris from the lysis of the organisms.

The nucleic acid hybridization step may now be carried out by simply spotting concentrated, detectably labeled nucleic acid probe reagent onto the binding surface, in an amount that will fit approximately within its boundaries, and will not overflow those boundaries. Suitable amounts typically range from about 5-30 microliters when the boundaries of the binding surface are in the order of 2-5 millimeters.

After allowing an appropriate amount of time to allow hybridization to take place the hybridization event may be detected through the detectable label of the probe.

A device for use in conducting limited volume nucleic acid hybridization comprises a substantially rigid inert support having a flat surface and at least one isolated nucleic acid binding surface adherently attached to a face of the support surface. The inert support may have an elongate or paddle shape. The isolated nucleic acid binding surface may be constructed of any material suitable to bind nucleic acids, and preferably cellulose, nylon, and positively charged nylon or nitrocellulose. The nucleic acid binding surface may be adhered to the inert support by any conventional means, especially hydrophobic adhesives. In the preferred embodiments, the inert surface is in the shape of a paddle as shown in the Figure. A pluralty of binding surface dots are positioned relative to one another along the longitudinal axis of the paddle handle, spaced at least about 1 mm from one another. The base of the paddle contains an area for labeling indicia.

A kit for identifying nucleic acid sequences comprises the device of the present invention and a vessel containing a detectably labeled nucleic acid probe reagent. The probe is specific for the sequence to be detected. In preferred embodiments, this sequence is specific to a particular microorganism. The nucleic acid probe reagent then contains at least one nucleotide sequence complementary to at least a portion of the nucleic acid within the microorganism. The present method may be used to identify any microorganism, as long as at least a portion of that microorganism's nucleic acid is available for hybridization to the probe reagent. One such microorganism is Neisseria gonorrhoeae. N. gonorrhoeae causes the sexually transmitted disease gonorrhea. Since infection caused by N. gonorrhoeae constitutes a reportable disease, it is usually required that confirmation tests be performed on suspect cultures. A confirmatory work-up may take the form of carbohydrate utilization tests, rapid fermentation tests, rapid tests based on amino peptidase activity or immunoassay techniques. The present method using nucleic acid hybridization probes provides advantages over all of these prior methods.

The genus Neisseria includes two species that are pathogenic for man: N. gonorrhoeae and N. meningitidis. Although N.gonorrhoeae is isolated from many patients with asymptomatic infections, it is always considered to be a pathogen. On the other hand, N. meningitidis may be isolated from the throat and nasopharynx in a small proportion of healthy individuals as well as patients with meningococcal disease. Other Neisseria species (i.e., sicca, lactamica, and cinerea), as well as Branhamella catarrhalis, rarely cause disease, but may be part of the normal flora and therefore must not be confused with gonococcus or meningococcus.

The method of the present invention is more particularly exemplified by the following examples, but is not to be considered limited thereby.

### EXAMPLES

### 1. Detection of N. Gonnorhoreae in a Culture:

Neisseria gonorrhoeae cultures were grown on a selective enriched media such as straight, Thayer Martin (TM), modified Thayer Martin (MTM), New York City (NYC) or Martin Lewis to insure the isolation of pathogenic Neisseria species. Inoculated media were incubated at 35-36°C in a humid 3-7% carbon-dioxide atmosphere until bacterial growth was evident (16-24 h). Cultures were inspected visually for colony morphology. Isolates were not older than 96 h (4 days). Suspect organisms were lifted from the plates by gently touching the bacterial colony with the flattened end of an applicator stick. The applicator was then used to dab the bacteria onto the test dot. A single 1mm colony was sufficient to perform this assay, so the test dot would not be overloaded. The bacteria was applied as evenly as possible and damage to the test dot, by scratching, tearing or excessive pressure, was avoided. The innoculated test paddle was then placed on a heat block at 37°C. A small bead (approximately 10 microliters) of lysis reagent (0.5 M alkali containing detergent) was added directly onto the test dot. The paddle was incubated for one minute at 37°C. The paddle was then removed from the heat block and placed in a neutralizing reagent (0.5 M Tris/HCl containing detergent) for one minute. The paddle was then returned directly to the heat block without delay, excess reagent was blotted off with absorbent paper using direct pressure.

A small bead (approximately 10 microliters) of the probe reagent (biotinylated N. gonorrhoeae specific probe and buffer containing 50% formamide, 10% hybridization enhancer, Triton X-100* and carrier DNA) was placed directly onto the test dot. The paddle was incubated for one minute at 37°C and blotted as before. Then, a small bead (approximately 10 microliters) of conjugate reagent (streptavidin-horseradish peroxidase complex containing 1% carrier proteins and stabilizers and preservative, 0.15% thimerosal) was added directly onto the test dot and incubated for one minute at 37°C. The paddle was removed from the heat block and excess reagent was tapped off. The paddle was then placed in a wash reagent to wash off excess probe and enzyme conjugate. The wash reagent contained a reducing agent and detergent. The paddle was soaked for two minutes and agitated intermittently. The paddle was returned to the heat block and blotted as before.
(* Triton X-100 is a registered trade mark.)

A small bead (approximately 10 microliters) of substrate solution (buffer containing hydrogen peroxide and 3,3′,5,5′-tetramethylbenzadine (TMB)) was added directly to the test dot. The paddle was incubated for one minute at 37°C. The paddle was removed from the heat block and placed in a stop reagent (1% sodium azide) for approximately 10 seconds. The paddle was then removed, blotted, and visually read to confirm the presence of N. gonorrhoeae. A positive test for N. gonorrhoeae produced a distinctive blue signal. The negative result ranged from no discernible signal to a faint blue background coloration.

Positive test samples were compared to positive and negative controls. Ideally, the positive control would be a fresh clinical isolate (less than 49 h old) that has been definitively identified as N. gonorrhoeae. Alternatively, a stock strain of N. gonorrhoeae may be obtained from a recognized depository, such as the American Type Culture Collection (ATCC), Rockville, Maryland 20850. Ideally, the negative control would be a fresh clinical isolate (less than 48 h old) of a confirmed non-gonorrhea causing Neisseria species (e.g. N. meningitidis). Alternatively, a stock strain could be attained from the ATCC.

This detection method was compared to the classic carbohydrate degradation test for identifying N. gonorrhoeae and other Neisseria species. A total of 361 genital and pharyngeal isolates were tested. The above-described method gave positive results for 174/174 confirmed N. gonorrhoeae isolates and gave negative results for 157/158 non-N. gonorrhoeae isolates.

### 2. PPNG Assay

The incidence of penicillinase producing Neisseria gonorrhoeae (PPNG) has increased significantly in the United States in the last couple of years. The penicillinase produced by Neisseria gonorrhoeae is of the TEM beta-lactamase type. DNA probes were synthesized using sequence information from the published sequence of the TEM-1 gene and these probes were labelled with biotinylated nucleotides using the enzyme terminal transferase. Using these probes, a rapid non-radioactive DNA probe assay was developed for the detection of PPNGs directly from primary culture. The assay followed the same format as the test for Neisseria gonorrhoeae described in Example 1 above. The assay took approximately 8-10 minutes to perform, and the final assay result was read visually. No false negative results were obtained with 43 PPNGs from primary culture plates.

### 3. Concurrent Detection of N. gonorrhoeae and PPNG

An assay according to Examples 1 and 2 is performed concurrently using a DNA probe for the nucleic acid of N. gonorrhoeae and a separate probe for PPNG. Sample bacteria are applied to the test dots. N. gonorrhoeae probe reagent is applied to certain of the dots and PPNG probe reagent to others. Negative controls may also be used. As in the previous examples, positive results give a blue coloration. This example demonstrates that a sample may be tested for more than one target nucleic acid on the same inert paddle support. However, care must be taken not to overflow the boundaries of the binding surfaces, so as to avoid cross contamination of the reagents.

### 4. Assays With Varying Detection Systems

In some cases the avidin/biotin detection system described above is not suitable because of the presence of endogenous biotin in the organisms to be tested (eg. Campylobactor). Other detection systems can be used in the assay format described.

Accordingly, a system was developed wherein the DNA probe was labelled with FITC and the final reaction products detected with an enzyme conjugated antibody specific for the FITC molecule.

Suspect organisms were lifted from culture plates by gently touching the bacterial colony with the flattened end of an applicator stick. The applicator was then used to dab the bacteria onto the test dot. A single 1mm colony was sufficient to perform this assay, so the test dot would not be overloaded. The bacteria was applied as evenly as possible and damage to the test dot, by scratching, tearing or excessive pressure, was avoided. The inoculated test paddle was then placed on a heat block at 37°C. A small bead (approximately 10 microliters) of lysis reagent (0.5 M alkali containing detergent) was added directly onto the test dot. The paddle was incubated for one minute at 37°C. The paddle was then removed from the heat block and placed in a neutralizing reagent (0.5 M Tris/HCl containing detergent and 2mg/ml Proteinase K) for one minute. The paddle was then returned directly to the heat block without delay, excess reagent was blotted off with absorbent paper using firm direct pressure.

A small bead (approximately 10 microliters) of the probe reagent containing FITC-labelled probe specific for the organism being tested (.02 pmole/»l in buffer containing 50% formamide, 10% hybridization enhancer, Triton X-100* and carrier DNA) was placed directly onto the test dot. The paddle was incubated for one minute at 37°C and blotted as before. Then, a small bead (approximately 10 microliters) of conjugate reagent (10»g/ml of anti FITC monoclonal conjugate to horseradish peroxidase, 200 mole Hepes, 1% BIgG, .025m EDTA, .02% Thimerasol, 1mM K₃[Fe(CEN)₆]) was added directly onto the test dot and incubated for one minute at 37°C. The paddle was removed from the heat block and excess reagent was tapped off. The paddle was then washed of excess probe and enzyme conjugate by soaking for two minutes with intermittent agitation in a conventional detergent wash. The paddle was then placed in a wash reagent containing detergent and 1M NaCl. The paddle was returned to the heat block and blotted as before.
(* Triton X-100 is a registered trade mark.)

A small bead (approximately 10 microliters) of substrate solution (buffer containing hydrogen peroxide and TMB) was added directly to the test dot. The paddle was incubated for one minute at 37°C. The paddle was removed from the heat block and placed in a stop reagent (1% sodium azide) for approximately 10 seconds. The paddle was then removed, blotted, and visually read. A positive test result produced a distinctive blue signal. The negative result ranged from no discernible signal to a faint blue background coloration.

The above experiment was repeated for the detection of several other species, namely Shigella, Salmonella, Campylobacter, and Neisseria gonorrhea. Again, positive test results produced a distinctive blue signal. Negative results ranged from a faint blue color to no color development.

### 5. Detection of specific DNA

The assay systems described above have also been used to assay for the presence of a specific sequence of DNA, whether or not that sequence is from a microorganism. In these systems, the sample DNA was added in solution to the test dot and allowed to dry on the dot. The assays were then performed as described in Examples 1 and/or 3. Detection of the specific sequence resulted in a distinctive blue signal.

### 6. Preparation of Isolated Nucleic Acid Binding Surfaces on an Assay Paddle Device

A sheet of Gene Screen Plus™ (New England Nuclear) was cut into strips. One end of each strip was attached to a membrane quide strip for insertion into a paddle assembly machine containing plastic paddles preprinted with labeling indicia. Glue (RTV 732 or Silastic medical adhesive silicone Type A- Dow Corning) was then applied to the plastic paddles by the use of a stencil with holes. The paddles were removed from the paddle mold, and within 20 minutes of application of the glue, three membrane dots were applied by punching holes from the membrane strips with a hole puncher. The punched holes, or "dots" adhered to the plastic paddle with glue that was applied with the stencil. The paddles were allowed to dry for a minimum of 10 hours. Each paddle contained three membrane dots, attached directly under numbering indicia and midway between the sides of the paddle. The dots were free of glue or any other foreign substance, so as not to interfere with any subsequent assay and the reading of final results. The resulting paddle assay device looked substantially like that depicted in the Figure.

### 7. Reagent Volumes

Several membrane dots, having varying diameters, were generated using the hole-punching procedure mentioned in Example 6. An attempt was made to pipette reagent volumes onto the dots to cover the surface, but not to overflow its boundaries. The results are as follows:

| Diameter of Membrane Dot in millimeters | Volume in Microliters (approx.) |
|---|---|
| 2.4 | 5 |
| 2.8 | 8 |
| 3.0 | 10 |
| 4.0 | 15 |
| 4.4 | 18 |
| 4.75 | 25 |

## Claims

1. A method, for identifying whether a sample contains a nucleic acid sequence specific for a particular microorganism, comprising:
a) applying a limited amount of said sample to an isolated nucleic acid binding surface having a width or diameter of from 2 mm to 5.0 mm, which is attached to an inert support;
b) treating said sample with a lysis reagent so as to release nucleic acids and so as to bind said nucleic acids to said support in substantially single-stranded form;
c) applying from 5 »l to 30 »l of a reagent containing at least one detectably-labeled nucleic acid probe, said probe being present in said reagent at a concentration of from 0.2 to 5,000 pM/ml, directly onto said binding surface under hybridization conditions, for a length of time appropriate to allow hybridization to take place;
d) washing said binding surface; and
e) detecting hybridization of the probe with the nucleic acid on the binding surface by means of the detectable label, thereby identifying whether said specific nucleic acid sequence is present.

2. The method of claim 1 wherein the nucleic acid probe has a nucleotide sequence complementary to at least a portion of nucleic acid from the microorganism.

3. The method of claim 1 or claim 2, wherein the volume of reagent applied to said binding surface is from 5 to 20 »l.

4. The method of any one of claims 1 to 3 wherein the detectable label is chemical, radioactive, fluorescent or chemiluminescent or is a light scattering particle.

5. The method of any one of claims 1 to 3 wherein the detectable label is an enzyme capable of causing a color change in a substrate solution or a ligand capable of binding to such an enzyme.

6. The method of claim 5, wherein the ligand is biotin.

7. The method of claim 6, further including after step (c) and before step (d), the step of applying an avidin or streptavidin enzyme complex to the binding surface, and then the subsequent step of applying an enzyme substrate solution to the surface.

8. The method of any one of claims 1 to 7 wherein the probe is DNA.

9. The method of any one of claims 1 to 7 wherein the probe is RNA.

10. The method of any one of claims 1 to 9 wherein at least two different detectably-labeled nucleic acid probe reagents are added to different binding surfaces in step (c) to assay for at least two different specific nucleic acid sequences in the same sample.

11. A device for carrying out the method of any one of claims 1 to 10, comprising:
a) a substantially rigid inert support; and
b) a plurality of isolated nucleic acid binding surfaces having a width or diameter of from 2 mm to 5 mm, said surfaces being adherently attached to a surface of the support and being separated from one another by at least 1 mm.

12. The device of claim 11, wherein the inert support has an elongate shape, and said isolated nucleic acid binding surfaces are located along a longitudinal axis of said support.

13. The device of claim 11 or claim 12 wherein the nucleic acid binding surfaces are constructed of cellulose, nylon, positively charged nylon, nitrocellulose or a mixture thereof.

14. A kit for carrying out the method of any one of claims 1 to 10, comprising:
a) a substantially rigid inert support;
b) a plurality of isolated nucleic acid binding surfaces having a width or diameter of from 2mm to 5 mm, said surfaces being adherently attached to a surface of the support and being separated from one another by at least 1 mm; and
c) a vessel containing a detectably labeled nucleic acid probe reagent, said probe being present in said reagent at a concentration of from 0.2 to 5,000 pM/ml, wherein the probe is specific for a microorganism to be identified and has a nucleotide sequence complementary to at least a portion of the nucleic acid within the microorganism.

15. The kit of claim 14 wherein said probe is labeled with biotin, iminobiotin or fluoroscein.

16. The kit of claim 14 or claim 15 wherein the probe is specific for N. gonnorhoeae.

## Patentansprüche

1. Verfahren zum Bestimmen, ob eine Probe eine für einen bestimmten Mikroorganismus spezifische Nukleinsäuresequenz enthält, umfassend:
a) das Auftragen einer begrenzten Menge der Probe auf eine isolierte Nukleinsäure-bindende Oberfläche mit einer Breite oder einem Durchmesser von 2 mm bis 5,0 mm, die an einem inerten Träger befestigt ist;
b) das Behandeln der Probe mit einem Lysereagenz, um Nukleinsäuren freizusetzen und um die Nukleinsäuren an den Träger in im wesentlichen einzelsträngiger Form zu binden;
c) das Auf tragen von 5 »l bis 30 »l eines mindestens eine nachweisbar markierte Nukleinsäure-Sonde enthaltenden Reagenzes, wobei die Sonde in dem Reagenz bei einer Konzentration von 0,2 bis 5000 pM/ml vorliegt, direkt auf die bindende Oberfläche unter Hybridisierungsbedingungen in einem Zeitraum, der für eine Hybridisierung ausreicht;
d) das Waschen der bindenden Oberfläche; und
e) das Nachweisen der Hybridisierung der Sonde mit der Nukleinsäure auf der bindenden Oberfläche mittels des nachweisbaren Markers, wodurch festgestellt wird, ob die spezifische Nukleinsäuresequenz vorliegt.

2. Verfahren Flach Anspruch 1, wobei die Nukleinsäure-Sonde eine Nukleotidsequenz aufweist, die zu mindestens einem Teil der Nukleinsäure des Mikroorganismusses komplementär ist.

3. Verfahren nach Anspruch 1 oder Anspruch 2, wobei das Volumen des auf die bindende Oberfläche aufgetragenen Reagenzes 5 bis 20 »l beträgt.

4. Verfahren nach einem der Ansprüche 1 bis 3, wobei der nachweisbare Marker chemisch, radioaktiv, fluoreszent oder chemilumineszent ist oder ein lichtstreuendes Partikel ist.

5. Verfahren nach einem der Ansprüche 1 bis 3, wobei der nachweisbare Marker ein Enzym ist, das in der Lage ist, in einer Substratlösung eine Farbänderung zu verursachen, oder ein Ligand ist, der an ein solches Enzym binden kann.

6. Verfahren nach Anspruch 5, wobei der Ligand Biotin ist.

7. Verfahren nach Anspruch 6, das darüber hinaus nach Schritt (c) und vor Schritt (d) den Schritt umfaßt, daß ein Avidin- oder Streptavidin-Enzym-Komplex auf die bindende Oberfläche aufgebracht wird, und dann den nachfolgenden Schritt umfaßt, daß eine Enzym-Substrat-Lösung auf die Oberfläche aufgebracht wird.

8. Verfahren nach einem der Ansprüche 1 bis 7, wobei die Sonde DNA ist.

9. Verfahren nach einem der Ansprüche 1 bis 7, wobei die Sonde RNA ist.

10. Verfahren nach einem der Ansprüche 1 bis 9, wobei mindestens zwei verschiedene Reagenzien von nachweisbar markierten Nukleinsäure-Sonden auf verschiedene bindende Oberflächen in Schritt (c) gegeben werden, um auf mindestens zwei verschiedene spezifische Nukleinsäuresequenzen in derselben Probe zu testen.

11. Vorrichtung zum Ausführen des Verfahrens nach einem der Ansprüche 1 bis 10, umfassend:
a) einen im wesentlichen steifen inerten Träger; und
b) eine Mehrzahl von isolierten Nukleinsäure-bindenden Oberflächen mit einer Breite oder einem Durchmesser von 2 mm bis 5 mm, wobei die Oberflächen an einer Oberfläche des Trägers haftend befestigt sind und voneinander mindestens 1 mm getrennt sind.

12. Vorrichtung nach Anspruch 11, wobei der inerte Träger eine längliche Form aufweist und sich die isolierten Nukleinsäure-bindenden Oberflächen entlang einer Längsachse des Trägers befinden.

13. Vorrichtung nach Anspruch 11 oder Anspruch 12, wobei die Nukleinsäure-bindenden Oberflächen ans Cellulose, Nylon, positiv geladenen Nylon, Nitrocellulose oder einem Gemisch davon gebildet sind.

14. Kit zum Ausführen des Verfahrens nach einem der Ansprüche 1 bis 10, umfassend:
a) einen im wesentlichen steifen inerten Träger;
b) eine Mehrzahl von isolierten Nukleinsäure-bindenden Oberflächen mit einer Breite oder einem Durchmesser von 2 mm bis 5 mm, wobei die Oberflächen an einer Oberfläche des Trägers haftend befestigt sind und voneinander mindestens 1 mm getrennt sind; und
c) einen Behälter, der ein Reagenz einer nachweisbar markierten Nukleinsäure-Sonde enthält, wobei die Sonde in dem Reagenz bei einer Konzentration von 0,2 bis 5000 pM/ml vorliegt, wobei die Sonde für einen zu identifizierenden Mikroorganismus spezifisch ist und eine Nukleotidsequenz aufweist, die zu mindestens einem Teil der Nukleinsäure in dem Mikroorganismus komplementär ist.

15. Kit nach Anspruch 14, wobei die Sonde mit Biotin, Iminobiotin oder Fluorescein markiert ist.

16. Kit nach Anspruch 14 oder Anspruch 15, wobei die Sonde für N. gonnorhoeae spezifisch ist.

## Revendications

1. Procédé pour identifier si un échantillon contient une séquence d'acide nucléique spécifique d'un microorganisme particulier, dans lequel:
a) on applique une quantité limitée dudit échantillon à une surface de liaison d'acide nucléique ayant une largeur ou un diamètre compris entre 2 mm et 5,0 mm, qui est attachée à un support inerte;
b) on traite ledit échantillon avec un réactif de lyse de manière à libérer les acides nucléiques et à lier lesdits acides nucléiques au dit support sous une forme sensiblement à un seul brin;
c) on applique de 5 »l à 30 »l d'un réactif contenant au moins une sonde d'acide nucléique marquée de façon détectable, ladite sonde étant présente dans ledit réactif à une concentration de 0,2 à 5000 pM/l, directement sur ladite surface de liaison dans des conditions d'hybridation, pendant une durée appropriée pour permettre à l'hybridation de s'effectuer;
d) on lave ladite surface de liaison; et
e) on détecte l'hybridation de la sonde avec l'acide nucléique sur la surface de liaison au moyen de la marque détectable, identifiant ainsi si oui ou non ladite séquence d'acide nucléique spécifique est présente.

2. Procédé de la revendication 1 dans lequel la sonde d'acide nucléique a une séquence nucléotidique complémentaire d'au moins une partie de l'acide nucléique du microorganisme.

3. Procédé de la revendication 1 ou de la revendication 2, dans lequel le volume de réactif appliqué à ladite surface de liaison est de 5 à 20 »l.

4. Procédé de l'une quelconque des revendications 1 à 3 dans lequel la marque détectable est chimique, radioactive, fluorescente ou chimioluminescente ou est une particule dispersant la lumière.

5. Procédé de l'une quelconque des revendications 1 à 3 dans lequel la marque détectable est une enzyme capable de provoquer un changement de couleur dans une solution de substrat ou un coordinat capable de se lier à une telle enzyme.

6. Procédé de la revendication 5 dans lequel le coordinat est la biotine.

7. Procédé de la revendication 6, incluant en outre après l'étape (c) et avant l'étape (d), l'étape d'application d'un complexe enzymatique d'avidine ou de streptavidine à la surface de liaison, puis l'étape ultérieure d'application d'une solution de substrat enzymatique à la surface.

8. Procédé de l'une quelconque des revendications 1 à 7 dans lequel la sonde est de l'ADN.

9. Procédé de l'une quelconque des revendications 1 à 7 dans lequel la sonde est de l'ARN.

10. Procédé de l'une quelconque des revendications 1 à 9 dans lequel on ajoute au moins deux réactifs sonde d'acide nucléique marqués de façon détectable à différentes surfaces de liaison dans l'étape (c) pour analyser au moins deux séquences d'acide nucléique spécifiques différentes dans le même échantillon.

11. Dispositif pour réaliser le procédé de l'une quelconque des revendications 1 à 10, comprenant:
a) un support inerte sensiblement rigide; et
b) plusieurs surfaces de liaison d'acide nucléique isolées ayant une largeur ou un diamètre de 2 mm à 5 mm, lesdites surfaces étant attachées de manière à adhérer à une surface du support et étant séparées l'une de l'autre par au moins 1 mm.

12. Dispositif de la revendication 11, dans lequel le support inerte a une forme allongée, et lesdites surfaces de liaison d'acide nucléique isolées sont situées le long d'un axe longitudinal dudit support.

13. Dispositif de la revendication 11 ou de la revendication 12 dans lequel lesdites surfaces de liaison d'acide nucléique sont construites en cellulose, nylon, nylon positivement chargé, nitrocellulose ou un de leurs mélanges.

14. Nécessaire pour réaliser le procédé de l'une quelconque des revendications 1 à 10, comprenant:
a) un support inerte sensiblement rigide;
b) plusieurs surfaces de liaison d'acide nucléique isolées ayant une largeur ou un diamètre compris entre 2 mm et 5 mm, lesdites surfaces étant attachées de manière à adhérer à une surface du support et étant séparées l'une de l'autre par au moins 1 mm; et
(c) un récipient contenant un réactif sonde d'acide nucléique marqué de façon détectable, ladite sonde étant présente dans ledit réactif à une concentration comprise entre 0,2 et 5000 pM/l, la sonde étant spécifique d'un microorganisme à identifier et ayant une séquence nucléotidique complémentaire d'au moins une partie de l'acide nucléique dans le microorganisme.

15. Nécessaire de la revendication 14 dans lequel ladite sonde est marquée avec de la biotine, de l'iminobiotine ou de la fluorescéine.

16. Nécessaire de la revendication 14 ou de la revendication 15 dans lequel la sonde est spécifique de N. gonorrhoeae.
